# EUROPÄISCHE PATENTSCHRIFT

(11) **EP 0 800 509 B1**
(45) Veröffentlichungstag und Bekanntmachung des Hinweises auf die Patenterteilung: **22.09.1999**
(21) Anmeldenummer: 95942680.0
(22) Anmeldetag: 16.12.1995
(51) Int. Cl.: C07C 253/30, C07C 255/24

(54) **VERFAHREN ZUR HERSTELLUNG VON ALIPHATISCHEN ALPHA,OMEGA-AMINONITRILEN**
PROCESS FOR PREPARING ALIPHATIC ALPHA,OMEGA-AMINONITRILES
PROCEDE DE PREPARATION D'AMINONITRILES-ALPHA,OMEGA ALIPHATIQUES

(30) Priorität: 27.12.1994 DE 4446895
(43) Veröffentlichungstag der Anmeldung: 15.10.1997
(73) Patentinhaber: BASF AKTIENGESELLSCHAFT, 67056 Ludwigshafen (DE)
(72) Erfinder: FLICK, Klemens, D-76863 Herxheim (DE); EBEL, Klaus, D-68623 Lampertheim (DE); SCHNURR, Werner, D-67273 Herxheim (DE); MELDER, Johann-Peter, D-67141 Neuhofen (DE); HARDER, Wolfgang, D-69469 Weinheim (DE)
(86) Internationale Anmeldenummer: EP9504987
(87) Internationale Veröffentlichungsnummer: WO9620167

(56) Entgegenhaltungen:
- EP-A- 0 161 419
- WO-A-92/21650
- WO-A-93/16034
- DE-A- 4 235 466
- US-A- 2 257 814

## Beschreibung

Die vorliegende Erfindung betrifft ein verbessertes Verfahren zur Herstellung von aliphatischen α,ω-Aminonitrilen durch partielle Hydrierung von aliphatischen α,ω-Dinitrilen bei erhöhter Temperatur und erhöhtem Druck in Gegenwart eines Lösungsmittels und eines Katalysators.

Die WO 92/21650 beschreibt die partielle Hydrierung von Adipodinitril zu 6-Aminocapronitril in Gegenwart eines Raney-Nickel-Katalysators und Ammoniak als Lösungsmittel mit einer Ausbeute von 60% bei einem Umsatz von 70%. Als Nebenprodukt entstehen 9% Hexamethylendiamin. Bei Verwendung von Methanol als Lösungsmittel und Zusatz einer Base, die nicht Ammoniak ist, kann bei einem Umsatz von 70% die Selektivität auf 89,5% gesteigert werden. Nachteilig an diesem Verfahren ist die geringe Standzeit des Katalysators.

In der US 2,257,814 und in der US 2,208,598 werden ebenfalls Herstellverfahren von 6-Aminocapronitril ausgehend von Adipodinitril beschrieben, wobei als Katalysatoren Raney-Cobalt, Eisen-, Nickel- und Cobalt-Katalysatoren auf verschiedenen Trägern eingesetzt werden. Nachteilig an diesen Verfahren sind die mit 50 bis 60% für technische Anwendungen zu niedrigen Selektivitäten.

Nach dem Verfahren der WO 93/16034 kann man die Ausbeute an Aminocapronitril dadurch steigern, daß man Adiponitril in Gegenwart von Raney-Nickel, einer Base wie Natrium-, Kalium-, Lithium- oder Ammoniumhydroxid und einer Übergangsmetall-Komplexverbindung, mit beispielsweise Eisen, Cobalt, Chrom oder Wolfram als Übergangsmetalle, und eines Lösungsmittels hydriert. Nach diesem Verfahren werden bei Umsätzen im Bereich von 45 bis 60% quantitative Ausbeuten an Aminocapronitril beschrieben. Nachteilig an diesem Verfahren ist die Aufarbeitung der zumeist toxischen Übergangsmetall-Komplexverbindungen aus den erhaltenen Reaktionsgemischen.

In der EP-A 161,419 wird die partielle Hydrierung von Adipodinitril unter Verwendung eines Rhodium-haltigen Katalsators auf einem Magnesiumoxid-Träger in Gegenwart von Ammoniak beschrieben. Bei einem Umsatz von 70% wird eine Selektivität von 94% erreicht. Nachteilig ist die aufwendige Herstellmethode der Rh/MgO-Katalysatoren (s. J. of Cat. 112 (1988), S. 145-156).

Aufgabe der vorliegenden war daher die Bereitstellung eines verbessertes Verfahrens zur Herstellung von aliphatischen α,ω-Aminonitrilen durch partielle Hydrierung von Adipodintril, das die zuvor genannten Nachteile nicht aufweist, insbesondere sollte ein Verfahren gefunden werden, indem die verwendeten Katalysatoren eine längere Standzeit im Vergleich zu denjenigen aus dem Stand der Technik aufweisen.

Demgemäß wurde ein Verfahren zur Herstellung von aliphatischen α,ω-Aminonitrilen durch partielle Hydrierung von aliphatischen α,ω-Dinitrilen bei erhöhter Temperatur und erhöhtem Druck in Gegenwart eines Lösungsmittels und eines Katalysators gefunden, indem man einen Katalysator verwendet, der erhältlich ist durch thermische Zersetzung einer Metallverbindung, ausgewählt aus der Gruppe, bestehend aus Metallcarbonylverbindungen, Metallsalzen von C₁-C₆-Carbonsäuren und C₂-C₆-Dicarbonsäuren und Metallkomplexen mit Diketonen, unter Erhalt des Metalls und/oder eines Metalloxids davon, mit der Maßgabe, daß man als Metall Nickel oder Cobalt verwendet.

Des weiteren wurde die Verwendung von durch thermische Zersetzung hergestellte Katalysatoren für die partielle Hydrierung von α,ω-Dinitrilen gefunden.

Unter den erfindungsgemäß in Betracht kommenden Metallverbindungen, Metallcarbonylverbindungen, Metallsalzen von C₁-C₆-Carbonsäuren und C₂-C₆-Dicarbonsäuren und Metallkomplexe mit Diketonen, bevorzugt 1,2- und 1,3-Diketone wie Acetylacetonat oder Dimethylglyoxim, setzt man bevorzugt Metallsalze der C₁-C₆-Carbonsäuren und C₂-C₆-Dicarbonsäuren ein, wobei hierunter auch ungesättigte Carbonsäuren und Dicarbonsäuren sowie Hydroxygruppen-haltige Carbon- und Dicarbonsäuren mitumfaßt werden sollen wie Maleinsäure, Milchsäure und Weinsäure. Insbesondere bevorzugt sind Nickel- und Cobaltsalze der Ameisensäure, Essigsäure, Propionsäure, Buttersäure, Valeriansäure, Capronsäure, Oxalsäure, Malonsäure, Bernsteinsäure, Glutarsäure, Adipinsäure und Maleinsäure, ganz besonders bevorzugt sind Nickelformiat, Cobaltformiat, Nickelacetat, Cobaltacetat, Nickel-Oxalat und Cobalt-Oxalat.

Die Metallsalze der Carbon- und Dicarbonsäuren sind entweder käuflich erhältlich oder beispielsweise gemäß J. of Phys. Chem. 68.4 (1964) S. 731-732 oder J. Am. Chem. Soc. 81 (1959), S. 2930-2933, herstellbar.

Die Herstellung der entsprechenden Katalysatoren aus den Metallsalzen der Carbonsäuren und Dicarbonsäuren ist in "Preparation of catalysts" V, 1991, Elsevier Science Publishers B.V., Amsterdam, S. 165-174 und Coll. Czech. Chem. Comm. 46 (1981), S. 1011-1022, beschrieben oder kann analog zu der dort beschriebenen Verfahrensweise durchgeführt werden, so daß sich nähere Angaben hierzu erübrigen.

Die thermische Zersetzung von Metallcarbonylen kann beispielsweise nach Verfahren gemäß DRP 511 564 oder FP 1,317,934 vorgenommen werden.

Die thermische Zersetzung von Metallkomplexen mit Diketonen führt man üblicherweise analog der thermischen Zersetzung der Salze der Carbonsäuren, jedoch in einer Wasserstoffatmosphäre, durch.

Im allgemeinen nimmt man die thermische Zersetzung in einem Temperaturbereich von 150 bis 500, bevorzugt von 180 bis 400, besonders bevorzugt von 190 bis 350°C vor, wobei die Temperaturauswahl sich im einzelnen daran orientiert, welche Ausgangsverbindung eingesetzt wird. Des weiteren kann die thermische Zersetzung sowohl in inerter (Stickstoff, Argon, Helium) als auch in reduzierender Atmosphäre (Wasserstoff) bei einem Druck im Bereich von 1 Pa bis 10 MPa, vorzugsweise von 100 Pa bis 1 MPa, besonders bevorzugt bei Atmosphärendruck, in an sich üblichen Reaktionsbehältern vorgenommen werden. Zur Herstellung von Trägerkatalysatoren führt man die thermische Zersetzung in Gegenwart des gewünschten Trägers oder einer Vorläuferverbindung des Trägers durch.

Bei der thermischen Zersetzung erhält man in der Regel in Abhängigkeit von der eingesetzten Metallverbindung, der Dauer der thermischen Zersetzung als auch der gewählten Temperatur und der Zusammensetzung der Gasphase aus den eingesetzten Metallverbindungen die entsprechenden Metalle oder Gemische der Metalle mit den jeweiligen Metalloxiden.

Der Gehalt an Nickel oder Cobalt im Katalysator hängt davon ab, ob ein Voll- oder ein Trägerkatalysator hergestell wird. Bei Trägerkatalysatoren liegt der Metallgehalt üblicherweise im Bereich von 1 bis 70, vorzugsweise von 10 bis 40 Gew.-%.

Des weiteren können die Katalysatoren außer Nickel bzw. Cobalt weitere Metalle, insbesondere Übergangsmetalle und Metalle der zweiten bis vierten Hauptgruppe des Periodensystems der Elemente enthalten, wobei Silber, Kupfer, Zink, Magnesium, Calcium und Eisen besonders bevorzugt sind. Die Herstellung erfolgt in der Regel durch gleichzeitige oder nacheinander durchgeführte thermische Zersetzung der entsprechenden Metallverbindungen, wobei man zweckmäßig eine Mischung von Metallverbindungen einsetzt, bevorzugt solche, in denen die Nichtmetall-haltigen Reste gleich sind. Beispielsweise kann man gemeinsam gefälltes Nickel- und Magnesiumoxid unter inerter Atmosphäre wie Stickstoff, Argon oder Helium oder reduzierender Atmosphäre mit Wasserstoff zu fein verteiltem Nickel auf Magnesiumoxid umsetzen (s. "Pre. of Cat. V, 1991, Elsevier, Amsterdam, S. 165-174, Coll. Czech. Chem. Comm. 46 (1981), S. 1011-1022).

In einer bevorzugten Ausführungsform erhält man Nickel-Katalysatoren gemäß dem Verfahren aus Ind.Eng.Chem. Vol.32, No.9, S. 1193-1199 (1940), indem man Nickelformiat in einer hochsiedenden Flüssigkeit erhitzt.

Analog zu dem Verfahren aus der Ind. Eng. Chem. Vo1.32, No.9, S. 1193-1199 (1940) kann man bevorzugt andere Nickel- und Cobalt-Katalysatoren in der Flüssigphase herstellen, wobei man unter Atmosphärendruck hochsiedende inerte Substanzen einsetzt, insbesondere hochsiedende gesättigte C₉-C₃₀-Kohlenwasserstoffe wie Weißöle und Paraffine, hochsiedende CₙH₂ₙ₊₃ N-Amine (mit n = 7 bis 25), hochsiedende CₘH₂ₘ₊₄ N-Diamine (wie m = 5 bis 35) wie Hexamethylendiamin, Polyamine wie Polyethylenimin und hochsiedende Alkohole der Zusammensetzung (OH)ₚC₇H_{2q+2-p} wie Octanol, Propandiol und Glycerol. Der Vorteil dieser Methode liegt in der Umhüllung des Nickels oder Cobalts sowie gewünschtenfalls weiterer Metalle mit der hochsiedenden Substanz, wobei die hochsiedende Substanz bei Raumtemperatur selbstverständlich auch ein Feststoff sein kann. Diese Umhüllung verhindert nach bisherigen Beobachtungen den Zutritt von Sauerstoff, so daß eine Passivierung des Katalysators unterbleibt und ein einfaches Arbeiten mit dem Katalysator erlaubt.

Als Ausgangsstoffe im erfindungsgemäßen Verfahren werden aliphatische α,ω-Dinitrile der allgemeinen Formel I

NC-(CH₂)ₙ-CN I

in der n eine ganze Zahl von 1 bis 10, insbesondere 2, 3, 4, 5 und 6, bedeutet, eingesetzt. Besonders bevorzugte Verbindungen I sind Bernsteinsäuredinitril, Glutarsäuredinitril, Adipinsäuredinitril ("Adiponitril"), Pimelinsäuredinitril und Korksäuredinitril ("Suberonitril"), ganz besonders bevorzugt Adiponitril. Nach dem erfindungsgemäßen Verfahren werden die vorstehend beschriebenen Dinitrile I in Gegenwart eines Lösungsmittels und gewünschtenfalls einer Base wie ein Hydroxid-der Alkalimetalle, insbesondere Lithiumhydroxid, Natriumhydroxid, Kaliumhydroxid, bevorzugt Lithiumhydroxid und den zuvor beschriebenen Katalysatoren partiell zu α,ω-Aminonitrilen der allgemeinen Formel II

NC-(CH₂)ₙ-CH₂-NH₂ II

hydriert, wobei n die vorstehend genannte Bedeutung hat. Besonders bevorzugte Aminonitrile II sind solche, in denen n einen Wert von 2, 3, 4, 5 oder 6 hat, insbesondere 4, d.h. 4-Aminobutansäurenitril, 5-Aminopentansäurenitril, 6-Aminohexansäurenitril ("6-Aminocapronitril"), 7-Aminoheptansäurenitril und 8-Aminooctansäurenitril, ganz besonders bevorzugt 6-Aminocapronitril.

Führt man die Umsetzung in einer Suspension durch, wählt man üblicherweise Temperaturen im Bereich von 30 bis 150, vorzugsweise von 50 bis 100, besonders vorzugsweise von 60 bis 90°C vor; den Druck wählt man im allgemeinen im Bereich von 2 bis 20, vorzugsweise von 3 bis 10, besonders bevorzugt von 4 bis 8 MPa. Die Verweilzeiten sind im wesentlichen von der gewünschten Ausbeute, Selektivität und dem gewünschten Umsatz abhängig; üblicherweise wählt man die Verweilzeit so, daß ein Maximum an Ausbeute erreicht wird, beispielsweise beim Einsatz von Adiponitril im Bereich von 30 min bis 10 h, vorzugsweise von 1 bis 5 h.

Bei der Suspensionsfahrweise setzt man als Lösungsmittel bevorzugt Ammoniak, Amine, Diamine und Triamine mit 1 bis 6 C-Atomen wie Trimethylamin, Triethylamin, Tripropylamin und Tributylamin oder Alkohole, bevorzugt Methanol und Ethanol, besonders bevorzugt Ammoniak ein. Zweckmäßig wählt man eine Dinitrilkonzentration im Bereich von 10 bis 90, vorzugsweise von 30 bis 80, besonders vorzugsweise von 40 bis 70 Gew.-%, bezogen auf die Summe von Dinitril und Lösungsmittel.

Die Suspensionshydrierung kann man diskontinuierlich oder, bevorzugt kontinuierlich, in der Regel in der Flüssigphase durchführen.

Man kann die partielle Hydrierung auch diskontinuierlich oder kontinuierlich in einem Festbettreaktor in Riesel- oder Sumpffahrweise durchführen, wobei man üblicherweise eine Temperatur im Bereich von 20 bis 150, vorzugsweise von 30 bis 90°C und einen Druck in der Regel im Bereich von 2 bis 30, vorzugsweise von 3 bis 20 MPa wählt. Erfindungsgemäß führt man die partielle Hydrierung in Gegenwart eines Lösungsmittels, bevorzugt Ammoniak, Amine, Diamine und Triamine mit 1 bis 6 C-Atomen wie Trimethylamin, Triethylamin, Tripropylamin und Tributylamin oder Alkohole, bevorzugt Methanol und Ethanol, besonders bevorzugt Ammoniak durch. In einer bevorzugten Ausführungsform wählt man einen Gehalt an Ammoniak im Bereich von 1 bis 10, bevorzugt von 2 bis 6 g pro g Adipodinitril. Bevorzugt wählt man dabei eine Katalysatorbelastung im Bereich von 0,1 bis 1,0 kg Adipodinitril/ 1*h. Auch hier kann man durch Veränderung der Verweilzeit den Umsatz und damit die Selektivität gezielt einstellen.

Nach dem erfindungsgemäßen Verfahren erhält man alpha,omega-Aminonitrile in in guten Selektivitäten, wobei die Katalysatoren gegenüber den Katalysatoren des Standes der Technik eine deutlich bessere Standzeit aufweisen. Die α,ω-Aminonitrile sind wichtige Ausgangsverbindungen zur Herstellung von cyclischen Lactamen, insbesondere 6-Aminocapronitril für Caprolactam.

### Beispiel 1 - Herstellung eines Katalysators

Eine Lösung von 0,5 mol Nickelnitrat, 0,5 mol Zinknitrat, 1 l Wasser, 1 mol Essigsäure und 1 mol Ammoniumacetat wurde auf 70°C erhitzt. Zu dieser Lösung wurden unter Rühren tropfenweise während 1 h 1,5 mol Oxalsäure in 2 1 Wasser gelöst gegeben. Die auf diese Weise erhaltene Suspension wurde bei einer Temperatur von 70°C für 24 h weitergerührt und dann filtriert. Der gewaschene Niederschlag wurde bei 80°C 16 h lang getrocknet. Der getrocknete Niederschlag wurde bei 350°C während 16 h einer Wasserstoffatmosphäre ausgesetzt. Das so behandelte Produkt wies ein Molverhältnis Ni/Zn von 1:1 auf.

### Beispiel 2 - Hydrierung von Adiponitril (ADN)

60 g ADN, 6 g des in Beispiel 1 erhaltenen Katalysators und 100 ml flüssigen Ammoniak wurden in einem Autoklaven auf 100°C in einer Wasserstoffatmosphäre (100 bar) erhitzt. Nach drei Stunden wurde die Reaktion abgebrochen. Die ADN-Umsetzung betrug 59 %. Es wurden die folgenden Ausbeuten (Angaben in Gew.-%, bezogen auf das Reaktionsgemisch) erhalten: 6-Aminocapronitril (ACN): 50 %, Hexamethylendiamin (HMD): 7 %, Azepane: 0,36 %, Azepine: 0,07 %, Hochsieder: 0,65 %.

### Vergleichsbeispiel

Beispiel 2 wurde wiederholt, mit dem Unterschied, daß ein Raney-Nickelkatalysator (Degussa, K 111 W) eingesetzt wurde. Die ADN-Umsetzung betrug 58 %. Es wurden die folgenden Ausbeuten (Angaben in Gew.-%, bezogen auf das Reaktionsgemisch) erhalten: 6-Aminocapronitril (ACN): 50 %, Hexamethylendiamin (HMD): 6,7 %, Azepane: 0,23 %, Azepine: 0,15 %, Hochsieder: 1,29 %.

## Patentansprüche

1. Verfahren zur Herstellung von aliphatischen α,ω-Aminonitrilen durch partielle Hydrierung von aliphatischen α,ω-Dinitrilen bei erhöhter Temperatur und erhöhtem Druck in Gegenwart eines Lösungsmittels und eines Katalysators, dadurch gekennzeichnet, daß man einen Katalysator verwendet, der erhältlich ist durch thermische Zersetzung einer Metallverbindung, ausgewählt aus der Gruppe, bestehend aus Metallcarbonylverbindungen, Metallsalzen von C₁-C₆-Carbonsäuren und C₂-C₆-Dicarbonsäuren und Metallkomplexen mit Diketonen unter Erhalt des entsprechenden Metalls und/oder eines Metalloxids davon, mit der Maßgabe, daß man als Metall Nickel oder Cobalt verwendet.

2. Verfahren nach Anspruch 1, dadurch gekennzeichnet, daß die Katalysatoren als weitere Komponenten Metalle, ausgewählt aus der Gruppe, bestehend aus Silber, Kupfer, Zink, Magnesium, Calcium und Eisen enthalten.

3. Verfahren nach Anspruch 1 oder 2, dadurch gekennzeichnet, daß man als α,ω-Dinitril Adipodinitril einsetzt unter Erhalt von 6-Aminocapronitril.

4. Verwendung eines Katalyators, erhältlich durch thermische Zersetzung einer Metallverbindung, ausgewählt aus der Gruppe, bestehend aus Metallcarbonylverbindungen, Metallsalzen von C₁-C₆-Carbonsäuren und C₂-C₆-Dicarbonsäuren und Metallkomplexen mit Diketonen, unter Erhalt des Metalls und/oder eines Metalloxids davon, mit der Maßgabe, daß man als Metall Nickel oder Cobalt verwendet, zur partiellen Hydrierung von aliphatischen α,ω-Dinitrilen bei erhöhter Temperatur und erhöhtem Druck in Gegenwart eines Lösungsmittels.

5. Verwendung nach Anspruch 4 zur Herstellung von 6-Aminocapronitril.

6. Verwendung nach Anspruch 4, dadurch gekennzeichnet, daß die Katalysatoren als weitere Komponenten Metalle, ausgewählt aus der Gruppe, bestehend aus Silber, Kupfer, Zink, Magnesium, Calcium und Eisen enthalten.

## Claims

1. A process for the preparation of aliphatic α,ω-aminonitriles by partial hydrogenation of aliphatic α,ω-dinitriles at elevated temperatures and superatmospheric pressure in the presence of a solvent and of a catalyst, which comprises using a catalyst which is obtainable by thermal decomposition of a metal compound selected from the group consisting of metal carbonyl compounds, metal salts of C₁-C₆-carboxylic acids and C₂-C₆-dicarboxylic acids and metal complexes with diketones to give the corresponding metal or a metal oxide thereof, with the proviso that the metal used is nickel or cobalt.

2. A process as claimed in claim 1, wherein the catalysts contain, as further components, metals selected from the group consisting of silver, copper, zinc, magnesium, calcium and iron.

3. A process as claimed in claim 1 or 2, wherein the α,ω-dinitrile used is adiponitrile, and 6-aminocapronitrile is obtained.

4. Use of a catalyst obtainable by thermal decomposition of a metal compound selected from the group consisting of metal carbonyl compounds, metal salts of C₁-C₆-carboxylic acids and C₂-C₆-dicarboxylic acids and metal complexes with diketones to give the metal or a metal oxide thereof, with the proviso that the metal used is nickel or cobalt, for the partial hydrogenation of aliphatic α,ω-dinitriles at elevated temperatures and superatmospheric pressure in the presence of a solvent.

5. Use as claimed in claim 4 for the preparation of 6-aminocapronitrile.

6. Use as claimed in claim 4, wherein the catalysts contain, as further components, metals selected from the group consisting of silver, copper, zinc, magnesium, calcium and iron.

## Revendications

1. Procédé de préparation d'α,ω-aminonitriles aliphatiques par hydrogénation partielle d'α,ω-dinitriles aliphatiques à température élevée et pression élevée en présence d'un solvant et d'un catalyseur, caractérisé en ce qu'on utilise un catalyseur qui peut être obtenu par décomposition thermique d'un composé métallique, choisi parmi le groupe constitué des composés carbonyle métalliques, des sels métalliques d'acides carboxyliques en C₁-C₆ et d'acides dicarboxyliques en C₂-C₆ et des complexes métalliques avec des dicétones, avec obtention du métal correspondant et/ou d'un oxyde de celui-ci, à la condition qu'on utilise comme métal du nickel ou du cobalt.

2. Procédé suivant la revendication 1, caractérisé en ce que le catalyseur contient, comme autres composants, des métaux choisis parmi le groupe constitué de l'argent, du cuivre, du zinc, du magnésium, du calcium et du fer.

3. Procédé suivant l'une des revendications 1 et 2, caractérisé en ce qu'on met en oeuvre, comme α,ω-dinitrile, de l'adipodinitrile avec obtention de 6-aminocapronitrile.

4. Utilisation d'un catalyseur, que l'on peut obtenir par décomposition thermique d'un composé métallique, choisi parmi le groupe constitué des composés carbonyle métalliques, des sels métalliques d'acides carboxyliques en C₁-C₆ et d'acides dicarboxyliques en C₂-C₆ et des complexes métalliques avec des dicétones, avec obtention du métal et/ou d'un oxyde de celui-ci, à la condition qu'on utilise comme métal du nickel ou du cobalt, pour l'hydrogénation partielle d'α,ω-dinitriles aliphatiques à température élevée et pression élevée, en présence d'un solvant.

5. Utilisation suivant la revendication 4, pour la préparation de 6-aminocapronitrile.

6. Utilisation suivant la revendication 4, caractérisée en ce que les catalyseurs contiennent, comme autres composants, des métaux choisis parmi le groupe constitué de l'argent, du cuivre, du zinc, du magnésium, du calcium et du fer.
